(19) Europäisches Patentamt
European Patent Office
Office européen des brevets

(11) EP 3 725 894 A1

(12) **EUROPEAN PATENT APPLICATION**

(43) Date of publication:
**21.10.2020 Bulletin 2020/43**

(21) Application number: **19169679.8**

(22) Date of filing: **16.04.2019**

(51) Int Cl.:
***C12Q 1/6858*** *(2018.01)* ***B01L 3/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Blod Diagnostik GmbH**
**28717 Bremen (DE)**

(72) Inventor: **Carstensen, Rebecca**
**28717 Bremen (DE)**

(74) Representative: **Meissner Bolte Partnerschaft mbB**
**Beselerstrasse 6**
**22607 Hamburg (DE)**

# (54) APPARATUS AND METHODS FOR NUCLEIC ACID TARGET ENRICHMENT, SUSPENSION AND QUANTIFICATION

(57) The invention relates to a method for detection and/or quantification of at least one nucleic acid sequence in a sample, comprising the steps of: (a) optionally measuring the nucleic acid concentration in the sample; (b) amplifying the at least one nucleic acid sequence by nested polymerase chain reaction using nucleic acids comprised in the sample as template to form a set of first amplicons comprised in a first reaction mixture; (c) optionally measuring the nucleic acid concentration in the first reaction mixture; (d) amplifying the at least one nucleic acid sequence by polymerase chain reaction using the set of first amplicons as template and using nucleotide primers which are specific for the at least one nucleic acid sequence and which are coupled to magnetic beads to form a set of bead-coupled second amplicons comprised in a second reaction mixture; (e) optionally isolating the magnetic beads from the second reaction mixture; (f) combining the magnetic beads from step (d) or (e) with a labeled probe, wherein the probe binds specifically to the nucleic acid sequence; (g) detecting and/or quantifying magnetic beads labeled with the probe; and (h) determining the presence and/or the amount of the at least one nucleic acid sequence in the sample based on the detection and/or quantification in step (g). Further, the invention relates to a device and system for carrying out this method.

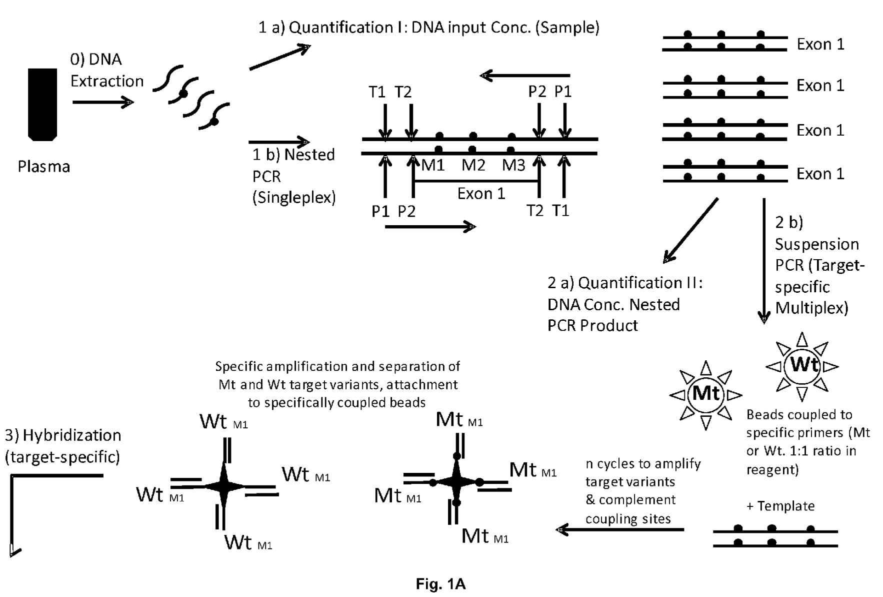


Fig. 1A

EP 3 725 894 A1

## Description

The present invention relates to an apparatus and methods for nucleic acid target enrichment, suspension and quantification that can be used for clinical diagnostic purposes, in particular in the field of personalized medicine.

Biomedical research and clinical diagnostics regularly require the detection of variations in individual genes, for example the detection of different variants of the same gene. The identification of particular variants of a gene can for example be used in the diagnosis of certain types of cancer and the assessment of appropriateness of various therapies (Sidransky, 2002, Nat Rev Cancer, 2, 210-219). The detection of underrepresented gene variations in a sample is often particularly difficult due to a lack of sensitivity and/or specificity of methods used in the art. A widely applicable diagnostic method would, further, have to be cost effective and, ideally, automatable.

A method that has aimed at overcoming these issues in the past is the BEAMing technology proposed by Dressman et al. (2003, PNAS, 100:15, 8817-8822) and US 9,360,526 B2. BEAMing according to Dressman *et al.* involves a first step of binding biotinylated oligonucleotides to streptavidin-coated magnetic beads; a second step of forming aqueous phase/oil phase-microemulsions statistically comprising inside aqueous droplets (a) one bead, (b) one copy of the template to be analysed and (c) the necessary components for a PCR; a third step of PCR cycling wherein the bead-bound oligonucleotides act as primers on the template; a fourth step of breaking the microemulsion and magnetically capturing the PCR-product-covered beads; a fifth step of sequence differentiation using sequence-specific probes labeled fluorescently and a sixth step of determining the amount of beads labeled in a particular manner by flow cytometry. While the BEAMing method has shown good results in the past, it also has certain limitations. The microemulsion generated in step 2 statistically comprises only one bead and template per droplet aqueous phase. However, a fraction of these compartments contained more due to a Poisson distribution and non-uniform aqueous compartment sizes (Dressman et al., 2003). Accordingly, the method might provide an inaccurate quantification of gene variants in the original sample. Moreover, the automation of the technique is challenging, in particular due to the use of the emulsion PCR.

There is, thus, still a need in the art for methods for a reliable, quantitative detection of gene variants that can be carried out in an automated manner by a correspondingly designed device.

## Description

This need is met by the present invention with a method according to claim 1, a device according to claim 11 and a system according to claim 12.

In a first aspect, the invention relates to a method for detection and/or quantification of at least one nucleic acid sequence in a sample, comprising the steps of:

(a) measuring the nucleic acid concentration in the sample;
(b) amplifying the at least one nucleic acid sequence by nested polymerase chain reaction using nucleic acids comprised in the sample as template to form a set of first amplicons comprised in a first reaction mixture;
(c) optionally measuring the nucleic acid concentration in the first reaction mixture;
(d) amplifying the at least one nucleic acid sequence by polymerase chain reaction using the set of first amplicons as template and using nucleotide primers which are specific for the at least one nucleic acid sequence and which are coupled to magnetic beads to form a set of bead-coupled second amplicons comprised in a second reaction mixture;
(e) optionally isolating the magnetic beads from the second reaction mixture;
(f) combining the magnetic beads from step (d) or (e) with a fluorescently labeled probe, wherein the probe binds specifically to the nucleic acid sequence;
(g) detecting and/or quantifying magnetic beads labeled with the probe; and
(h) determining the presence and/or the amount of the at least one nucleic acid sequence in the sample based on the detection and/or quantification in step (g).

The steps preferably carried out in the method are graphically and exemplarily displayed in Fig. 1.

The method of the invention is suitable for the detection and/or quantification, preferably both, of at least one nucleic acid sequence in a sample. The nucleic acid sequence can be a DNA or RNA sequence, such as an mRNA sequence, wherein DNA sequences are particularly preferred. The sequence may, e.g. be a genomic DNA sequence.

The nucleic acid sequence can be a prokaryotic, eukaryotic or artificial sequence. The method is particularly suitable for the detection and/or quantification of eukaryotic sequences, for example, mammalian sequences. The sequence may, e.g. be a human sequence, for example a human genomic DNA sequence.

The nucleic acid sequence will usually be a specific sequence. This means that the sequence occurs only once in the genome and is, in particular, not a part of a sequence repeat. Thus, the method of the invention can be used to identify the amount or the presence of a particular gene variant, allele, virus genome or the like in a sample.

Advantageously, the method according to the invention allows for the simultaneous detection of more than one nucleic acid sequence in a sample. Therefore, it is possible, for example, to detect at the same time a wild type sequence as well as a mutant sequence thereof. The mutant sequence may, for example, comprise an insertion, deletion, inversion or replacement of one or more bases. In a preferred embodiment of the invention at least two nucleic acid sequences are detected and/or quantified, e.g. a mutant and a wild-type sequence of the same gene or gene fragment. However, it is also possible with the methods disclosed herein to detect and/or quantify 3 or more, 4 or more or 5 or more nucleic acid sequences at the same time. The number of nucleic acid sequences that can be detected at the same time is only limited by the number of distinguishable probe labels. "Simultaneous" and "at the same time" mean that the respective number of different nucleic acid sequences can be detected within the same sample.

The sample used is preferably not an unpurified biological sample, but a purified nucleic acid sample without major impurities. Therefore, the methods described herein may also comprise a first step of isolating and/or purifying the nucleic acid sequence or sequences from or within the biological sample. Methods for nucleic acid purification or enrichment are known in the art and include e.g. the precipitation of the nucleic acid from a biological sample under high salt conditions, removing the supernatant and re-suspending the nucleic acids in a suitable buffer. Usually, the sample used in the method disclosed herein will comprise buffers and/or salts which are required for solving the nucleic acids to be analyzed.

***(a), (c) - measuring nucleic acid concentration***

In steps (a) and (c), the nucleic acid concentration in the sample can be determined. Methods which are suitable for determination of nucleic acid concentration are known in the art. In the context of the invention, it is particularly preferred to use microvolume spectrometry for the measurement. This technique has the particular advantage that only minor amounts of the sample are required for a measurement, preferably 10 μl or less. It can be seen in Fig. 1A, that the required microvolume of the sample to be analyzed is removed from the sample for the purpose of determining the nucleic acid concentration. Instruments which are able to perform such measurements are commercially available and include NanoDrop™ One (ThermoFisher) and others.

***Sequence-specific amplification and hybridization steps***

The method according to the present invention involves several amplification and hybridization steps which are sequence-specific. These steps act as checkpoints which reduce the rate of potential false positives as well as false negatives considerably. In particular, the method comprises two sequence-specific amplification steps (b) and (d) and one sequence-specific hybridization (f) step. The second sequence-specific amplification step (d) and the sequence-specific hybridization step (f) are specific for a particular region of interest (RoI) within the nucleic acid sequence. In other words, these steps (d) and (f) are specific for the particular region of interest, while the first sequence-specific amplification step (b) is specific for a sequence adjacent to the region of interest, thus, ensuring that variants of the RoI will also be amplified in said first amplification step (b). Such a "region of interest" (RoI) is a region, i.e. a subsequence, in which nucleic acids variation(s) occur; for example, a subsequence in which two nucleic acid sequences to be analyzed in the method of the invention differ. The subsequence, i.e. region of interest, can have a length of 1, 2, 3, 4, 5, 6, 7, 8, 9, 10 or more nucleotides.

***(b) - first amplification step***

In the first sequence-specific amplification step - step b) - the at least one nucleic acid sequence is amplified by nested polymerase chain reaction (PCR) using nucleic acids comprised in the sample as template to form a set of first amplicons, preferably by singleplex nested PCR. In this step it would be possible to amplify more than one nucleic acid sequence using the same primers. For example, the same primers could be used in a nested PCR to amplify a wild type sequence as well as a variant sequence thereof. The resulting first set of amplicons would then comprise the wild type sequence as well as the variant sequence, or, in other words, a first nucleic acid sequence and a second nucleic acid sequence. For this purpose, the respective primer pairs would be designed to bind to sequences (primer binding sites) surrounding the nucleic acid sequence position(s) of interest (RoI), i.e. the position(s) in which the two nucleic acid sequences differ. These positions may be relevant for a certain disease or health state. The primer binding sites may be directly adjacent to the RoI, or distanced from the RoI by 1 or more, 5 or more, preferably 10 or more, more preferably 20 or more amino acids. These distances refer to the second, i.e. "inner" primer pair of a nested PCR. The person skilled in the art is able to design suitable primers and locate suitable primer binding sites surrounding the RoI.

The person skilled in the art knows suitable nested PCR techniques and supplies needed for this method. In a nested PCR two sets of primers are used to amplify a target region of interest (RoI) in two successive PCR runs. The second PCR set is intended to amplify a secondary target within the product of the first PCR run. It will be understood

that both primer pairs of the nested PCR will bind surround the RoI, thus, ensuring that the RoI is fully amplified.

***(d) - second amplification step***

In the second sequence-specific amplification step - step d) - the at least one nucleic acid sequence is amplified by polymerase chain reaction using the set of first amplicons as template and using nucleotide primers which are specific for the at least one nucleic acid sequence and which are coupled to magnetic beads to form a set of bead-coupled second amplicons comprised in a second reaction mixture. In contrast to the aforementioned BEAMing technology, the present method does not include a PCR reaction carried out in an emulsion (e.g. in an aqueous phase - oil phase emulsion). Instead, the PCR, in particular the PCR in step d), is carried out in suspension. Thereby, the process is simplified and can be easily automated. The "breaking" of an emulsion is no longer required. This is advantageous because the creation and breaking of the emulsion, i.e. the phase separation between the polar and non-polar phases, is a complex process which would never happen without artificial, sensitive intervention by an emulsification mechanism. Moreover, the input to the emulsification and the output, i.e. how many microcompartments in relation to how many template molecules are generated, has to date not been sufficiently studied.

The second PCR step (d) uses nucleotide primers which are coupled to magnetic beads. Thereby, the second set of amplicons, thus, generated can be purified magnetically and differentiated by their respective fluorescent label (according to emitted wavelength detected by corresponding fluorescence channels of the flow cytometer). This is exemplarily shown in Fig. 2, Fig. 3 and Fig. 4.

Suitable magnetic beads are known in the art and include MyOne Dynabeads by Thermo Fisher Scientific. The beads can be labeled with nucleotide primers as described in Novex life technologies "User guide. Dynabeads Antibody Coupling Kit" (2012*).* Preferably, the beads have a low molecular weight, for example achieved by a low density and/or gaseous inclusions. Moreover, the beads have preferably stable surface properties, ensuring no or limited corrosion and no change in roughness of surface, and good colour stability, abrasion resistance and no or limited solubility in aqueous solutions. The beads can, further, have

(I) a defined diameter, preferably a diameter from 0,1 to 2 $\mu$m;
(II) a defined number of coupling sites, preferably 1 or more, 2 or more, 5 or more, or 10 or more, on their surface;
(III) optionally magnetic properties;
(V) a size distribution with CV $\leq$ 5 % (in diameter); and
(VI) an iron content of $\leq$ 30 %.

The nucleotide primers used in this second PCR reaction will be specific for a sequence within the first set of amplicons. Preferably, the primers are specific for a region that is inside of the region of interest. For example, in case a wild type and mutant nucleic acid sequence are analyzed at the same time, primers will bind to a sequence differing between these two sequences. In other words, in case more than one nucleic acid sequence is quantified and/or detected in the method of the invention, the nucleotide primers used in step (d) are suitable to distinguish between the two or more nucleic acid sequences. Each primer pair will, thus, specifically bind to one of the nucleic acid sequences and will not or at least substantially not bind to any of the other nucleic acid sequences to be detected and/or quantified in the method. As is known to the person skilled in the art, the sequence specificity of the primers can be verified experimentally or with the help of bioinformatics tools, preferably both. The primers will usually bind to a sequence in which the two or more nucleic acid sequences differ, i.e. to the RoI. The PCR of step d) is, thus, preferably a multiplex PCR.

***(e) - isolating beads***

After the second PCR step (d), the magnetic beads are preferably isolated from unbound template and other compounds which are not specifically bound to the beads. Before the hybridization step (f) is carried out, the beads can be isolated from the second reaction mixture. This can be achieved by excertion of magnetic forces, e. g electromagnetic force, on the beads in such a manner that they are drawn to a particular end of the vial or plate in which the reactions are performed. Subsequently, the remaining compounds can be carefully removed from the vial or plate. Then the magnetic force is removed and a new buffer can be added to the beads. It will be appreciated by the skilled person that this step can be considered to be a washing step. The step may be repeated if necessary.

***(f) - hybridization step: combining beads and probe***

The third sequence-specific step is specific for the region of interest (RoI) within the nucleic acid sequence, for example for the position in which a particular mutation can occur or in which a wild type and mutant sequence differ (see above). This step is, thus, used to differentiate between nucleic acid sequences, i.e. to identify a specific sequence

and/or to differentiate between two or more nucleic acid sequences.

In this step - step (f) - the magnetic beads from step (d) or (e) are combined with a fluorescently labeled probe that binds specifically to the nucleic acid sequence. In preferred embodiments, the fluorescently labeled probe binds to one or more positions in the region of interest in the nucleic acid sequence. The probe will be designed in a manner that ensures that the absence of a particular nucleic acid in this or these positions ensures that the probe can no longer bind to the nucleic acid sequence. In other words, in case more than one nucleic acid sequence is quantified and/or detected in the method of the invention, the probes used in step (f) are suitable to distinguish between the two or more nucleic acid sequences. Each probe will, thus, specifically bind to one of the nucleic acid sequences and will not or at least substantially not bind to any of the other nucleic acid sequences to be detected and/or quantified in the method. As is known to the person skilled in the art, the sequence specificity of the probes can be verified experimentally or with the help of bioinformatics tools, preferably both. The probes will usually bind to a sequence in which the two or more nucleic acid sequences differ, i.e. to the RoI. The skilled person is able to design such probes using standard computer programs.

Generally, a probe as well as the aforementioned primers comprise sequences that are complementary to the sequences to which they shall bind, i.e. their binding sequences. Usually, the probes are nucleic acids or fragments thereof and they are able to hybridize to their binding sequences. Each probe is preferably a single nucleic acid or fragment thereof.

The probes are labeled with a fluorescent label (fluorophore) that is suitable for use in flow cytometry, i.e. can be detected during flow cytometry. Suitable fluorescent labels are known in the art. It is also possible to label the probe with quantum dots or isotopes. Preferably, the label is attached covalently to the probe.

***(g) - detecting and/or quantifying beads***

In a subsequent step (g), the magnetic beads labeled with the probe are detected and/or quantified, preferably by means of the label, e.g. the fluorescent label, attached to them. Specifically, the number of beads which are attached to the second amplicon and were, thus, labeled with the fluorescently labeled probe in step (f), is detected by detection of the fluorescent signal. In other words, the number of hybridized, fluorescently labeled target sequences coupled to beads ("extended beads") is detected.

In cases in which more than one nucleic acid sequence is detected and/or quantified, the reaction mixture at this stage will comprise bead populations which are labeled with different probes and, thus, different labels. Each population can be detected and/or quantified separately according to the principles described herein.

Preferably, detection and quantification can be done by flow cytometry. For example, the detection range of the fluorescence detection channels encompasses the fluorescent emission signal (expressed as wavelength [nm]) of the probe specific for the nucleic acid sequence, e.g. the wild type or mutant probe. In cases in which more than one nucleic acid sequence is detected and/or quantified, the flow cytometric detection method allows a clear separation of several fluorescence signals by distinct wavelength definition of the respective probe label (fluorescent dye) and discrete channel detection range. The skilled person knows suitable flow cytometric detection techniques and will *inter alia* ensure that no overlaps in the detection ranges of different labels each associated with a different probe occur. The graphical output of a flow cytometric analysis according to the present invention is exemplarily displayed in Figs. 2, Fig. 3 and Fig. 4.

***(h) - Determining the presence and/or the amount of the at least one nucleic acid sequence***

Based on said detection in step (g), the presence and/or the amount of the at least one nucleic acid sequence in the sample can be determined in step (h). The skilled person is generally aware of methods which are suitable for said determination e.g. using flow cytometric output. The state of the art regarding suitable analysis methods and problems which they encounter will be briefly discussed herein below. Subsequently, the analysis proposed by the present invention will be explained.

*State of the art*

According to the present invention, calculation of the result values (i.e. presence and/or amount of the at least one nucleic acid sequence) uses validation parameters from two inbuilt measurement methods. The analysis concept results from a bottom-up approach as required by a *failure mode and effect analysis* (FMEA), and it considers statistical and mathematical requirements to ensure that factors, such as statistic sample size, do no result in distorted or false parameter results, or test results. The statistic sample size must be taken into consideration during the analysis to ensure a "true statement" regarding the data point distribution of flow cytometry data plots. It is known in the art that outliers can significantly distort parameters such as CV, RCV, mean and median which are commonly used in data analysis (Shapiro, Howard. Practical Flow Cytometry. 4th ed. NewYork: Wiley-Liss, 2003: pp. 231-236.Print.; CV: The Coefficient of Variation is a normalized Standard Deviation. CV = StdDev/Mean; RCV = Robust CV. The robust CV is not as skewed

by outlying values as the CV). This problem is addressed herein by specific case handling (of different distribution and/or stray behaviours) for different flow cytometry applications. Nevertheless, haematological applications commonly handle much larger particle [cell] populations, and hence bigger statistical sample sizes. Therefore, they run little to no risk of failure when using statistical functions as an absolute measure for population validation.

In bead-based technologies, problems resulting from low statistic sample size have to be taken into consideration to ensure that an algorithm is conceptualized to consider Poisson distributions, where using absolute values does not allow pattern recognition by statistic means. In other words: a CV or RCV as such are only reliable with sufficiently high number (n) of beads.

This can be deduced as follows: The CV does not consider outliers; it is therefore more susceptible to Poisson distributions, in particular with decreasing or low statistic sample size. This can be compensated to a certain degree by adjusting the method yielding the robust CV (RCV), which considers the inter quartile range to eliminate outliers. For a normal (Gaussian) distribution, Shapiro (Shapiro, H. Practical Flow Cytometry. 4th ed. NewYork: Wiley-Liss, 2003: p.236) suggests its calculation as follows:

$$RCV = 0{,}75 \text{ x i.q.r. (interquartile range)/median}$$

In this calculation, 75% of points lie within 0,68 SD of the mean. The equation can be modified to consider 95% of data points which are located within 1,65 SD of mean. This provides the following equation:

$$RCV = 0{,}95 \text{ x i.q.r./median}$$

But the remaining 95% percent of particles in the i.q.r. are still dependent on their statistic sample size for reliable evaluation. A cytometer only counts complete particles or cells, not half particles. Therefore, only integers (positive integers; e.g. 1,2,3,4,5,...n) can result as a particle count (number), each of them described by at least x- and y-coordinate for position. Given this precondition, it is only possible to start calculating a robust CV for 95% starting from 20 particles upwards at all: 19 particles/20 particles = 0,95.

However, "true" 95% are only evaluable when ≥ 100 particles have been counted in the target region to which the statistic function is applied, where 95 particles correspond to 95 %. In other words: 20 beads only provide 1/5 (0,2) of the statistic significance, while 50 beads already improve it to ½ (0,5) for a normal distribution. The effect of each single bead (position) on the value expression is thus 5 times higher for a size of 20, and 2 times as high for a sample size of 50 particles, when compared to a population of n ≥ 100. Thus, 100 particles provide the starting point of equivalence between the true data point distribution pattern and the statistic significance of the parameter result for CV and RCV.

These are mostly used to judge the stray behaviour of a population and position truth of the analyte molecules. Finally, such considerations are not only important to allow automated distribution recognition, but to ensure that test result analysis is aligned with QC; most importantly to prevent that test parameter values influenced by low statistic sample size are mistaken as performance error of the flow cytometer and instrument calibration.

Criteria for assessing analytical performance of a quantitative IVD are defined by the CLSI guidelines (EP17-A2, 2012) and by the IVDR (745, 2017) assessing the Limit of Blank (LoB), Limit of Detection (LoD) and Limit of Quantification (LoQ), which are critical "when detection of extremely small amounts of a measurand is necessary to define disease states, screen for presence of disease [...]. Knowledge of these estimates also is important for laboratory measurement procedures that measure circulating levels of tumor markers [...] and other biomarkers for which low results separate subjects into different disease or exposure categories" (CLSI EP17-A2, 2012, p. vii). This is particularly important when attempting to monitor the mutation status and quantity throughout gene-targeted therapy.

Since sample quality and experimental runs may be subject to variability, the likelihood to run into performance-related misinterpretation remains in case the issue of low statistic sample size is not assessed properly for potentially highly sensitive methods. This can either lead to false conclusions on the concordance or discordance of the result with regard to the reference method and/or sample type, or compromise sensitivity (Limit of Detection; LoD) claims that are not proven by evidence with regards to the (flow cytometry) data evaluation algorithms.

If e.g. the mutant fraction would be determined for an LoD of 0,02% based on a number of 20 000 extended beads, this would correspond to a mutant population size of 4 mutant beads (!), based on 30 000 beads to 6 beads - both not a measure for justifying a positive call in terms of a "population" - and finally 100 000 extended beads to 250 000 extended beads (EB) to make a positive call with 20 to 50 mutant signals (beads) for a fraction value respectively.

Thus, if the minimum EB count would not be determined to take these effects into consideration, the assay could simply not be capable of making a positive call for lower extended bead counts, and potentially yield false negatives. In that case, any sensitivity/LoD claim would only be a proof of concept for a successful assay run and high DNA concentration in the biological sample.

On the other hand, in cases in which the EB count is too low and the mutant bead count is by coincidence high enough, this might lead to false mutant fraction values distorted towards a high fraction, and hence false positives.

As a consequence, the combination of a consistently applicable LoD claim and significant statistic sample size to interpret the goodness of population phenotype require high EB yields in a bead-based assay for nucleic acid detection, and mitigation strategies beyond statistical measures where sample sizes of the mutant bead population are small (between 20 and 99 beads).

In practice presently few assays will be found which meet the expected best case scenario, however, it is nevertheless the task of the present result evaluation concept according to the invention to differentiate between cases in which evaluation algorithms may leave room for experimentally caused variation, and cases in which one must judge the results conservative. The issue of increasing detection threshold of a method whilst maintaining sensitivity (LoD) to avoid false positives has also been addressed in discussions on the BEAMing, NGS and real time PCR-based technologies (Garcia et al, 2018. "Oncotarget". Vol. 9 (No. 30): p. 21125-21126).

Another aspect to be aware of during result evaluation is the inclusion of suitable controls: Since PCR-based assays also rely on the template input (sample quality criterion) performance-wise, it would not make sense to create a control that judges the sample quality with regard to template input starting out from the assay itself, meaning that it is read out as another test parameter of the actual assay. This could result in what is called a "systematic error" (FDA, 2015, Elemental Analysis Manual for Food and Related Products, Version 2.0). Therefore, the present invention assesses these problems by assay design and data analysis algorithms and includes an external reference method for sample quality and initial amplification control.

*Sample and Data Analysis Concept according to the invention*

The present invention involves two different measurement methods, namely the determinations of DNA concentration and flow cytometric analysis. The first serves as an internal reference for sample quality and successful target enrichment which are a precondition for the reliable target detection. The latter evaluates the presence and quantity of the actual target (analyte/s).

Sample Quality Validation and In-process Amplification Control using (microvolume) spectrometry includes preferably (a) the determination of DNA concentration in the biological sample, e.g. a human blood plasma sample, and (b) the determination of the DNA concentration for assessment of the success of the nested PCR amplification.

*Target detection and quantification using flow cytometry*

In addition to the internal controls, external controls (control reagents) are preferably tested in parallel to the samples to be analyzed. The controls include positive controls (= containing WT and MT sequence), negative controls (containing the WT sequence only) and no template controls (no DNA template; no target yield expected). The test result is compared with the external controls for result validation.

For the concept of "internal control" as described in other PCR-based detection methods for e.g. mycological infection testing (*Mycobacterium Tuberculosis,* FDA Draft Guidance, 2011), of bacteria in food (FDA qPCR method for the Detection of *Salmonella* in Papaya, 2011), and as required e.g. per regulation 862.1660 for "Internal Polymerase Chain Reaction Control, Not Assay Specific" (Regulation Description: Quality control material (assayed and unassayed). Last upate 4th January 2019), it is pointed out that this prior art type of internal control is mainly dedicated to the purpose of preventing false-negative results due to failure of amplification. However, the problem underlying these PCR-based assays and detected analytes differs from that underlying technologies such as BEAMing and the present invention. It is risky to choose such an internal control design for the following reasons:

The nature of the analyte and detection method differ between PCR-based methods aiming to detect the presence and quantity of a particular analyte/target and bead-based PCR assays, e.g. for determination of tumor status and quantity by target variants (sequence variations). While an assayed or unassayed Internal Control may be included in PCR-based assays since it is unlikely that this will disturb the detection of the actual biological sample, there are several risks connected to the use of such a normal control in a bead-based PCR assay, in particular one associated with flow cytometry. Firstly, the cytometer and configuration is ideally set up in such way that a clear and reliable distinction of all detected colours (wavelength ranges or values for a dye in a fluorescently labeled probe) is ensured and that probes (dyes) in combination with detection channels are selected in discrete manner, meaning that there is by system design no overlap in detection of a signal in a non-intended fluorescence channel (compare Fig. 2). Otherwise, falsified counts and hence, result interpretations could occur. Such a control concept is with regard to flow cytometer configuration preferably carried out with a minimum of a distinct channel and colour (dye) for the internal control, because the quadrants QI and QIII of plot 3 according to the present invention (see Figs. 2 and 4) are required to remain empty in order to judge the quality of the WT and MT fraction counts and values. In the worst case, noise could be confused with the internal control population, leading to false validation or invalidation.

Moreover, if an "assayed" internal control is designed, its successful or unsuccessful amplification cannot necessarily be correlated with the biological sample amplification as such. It may, from a risk perspective, be possible that either the sample is perfectly fine and the control did not work - which would mean delay due to unjustified invalidation - or the IC is amplified, but the sample is not amplified successfully or in unsufficient manner. So despite of the fact that we know the IC worked, we get no information on why exactly the sample was not amplified.

And furthermore, a readout of an assayed IC means in this case - both for BEAMing and the present invention - that a second PCR step would follow. Thus, the true cause of failure cannot be deduced. Where bead-based PCR assays incorporate several PCR steps and a fluorescent-labeling procedure (by hybridization and/or base-extension), each of them can be the source of error. The latter case is again a result of different possible causes, including sample and reagent quality. Instrument-related failures are not mentioned here because they should be covered by preceding QC measurement, with the only exception of high noise levels in individual runs. But these must not necessarily be attributed to the instrument, it may also result from the quality of the reaction aliquot which is read out.

Therefore, the present invention proposes preferably the use of two inbuilt internal control concepts as risk mitigation by design, which do a) not interfere with the actual sample result interpretation, b) provide a reliable reference measure for sample quality and target enrichment (amplification) and c) confirm the presence and expected quantity of the actual target variants in the sample.

*Explanation of individual test or control result evaluation*

Calculation of hypothetically expected target yield based on PCR amplification for DNA. Amplicons of the first 2 cycles are only doubled, while from the 3rd cycle onwards, an exponential amplification is postulated as hypothetical mathematical model for PCR. Further deviation may result from consumption of primers, but an approximation can be assumed (Reinard, Thomas. Molekularbiologische Methoden 2.0. 2nd ed. Stuttgart: utb, 2018:, 85 f.).

PCR amplification (hypothetical):

II

II II

II II II II

II II II II   II II II II

Amplification[1)]: $(2^n - 2n)*x$

Where n = number of cycles,

2n = amplicons of first 2 cycles,

x = original DNA copy number,

[1)] assuming exponential amplification

| Test Parameter | Unit / Formula / Function | Value and Interpretation |
|---|---|---|
| ***Reference Measurements: Sample Quality and Amplification Control*** | | |
| DNA concentration (Template input) | ng/µl or GE/µl [converted] Conversion can be done using the formula: (*6,023 x $10^{23}$ (copies $mol^{-1}$) x conc. of standard (g $\mu l^{-1}$)) / MW (g/mol) And by establishing a standard curve for DNA concentration determination (Smith, 2006, Environ Microbiol, 8(5),804-815).<br>*Avogadro's number | DNA concentration can be evaluated to determine whether it is sufficiently high to allow the required statistic sample size for (inter-) test evaluation and comparison; optionally, the DNA content can be used to calculate mutant fraction as absolute value.<br>*The initial measurement of DNA concentration is, thus, able to verify whether the sample quality is sufficient for running the assay and obtain representative results.* |

(continued)

| Test Parameter | Unit / Formula / Function | Value and Interpretation |
|---|---|---|
| ***Reference Measurements: Sample Quality and Amplification Control*** | | |
| PCR Product (DNA) Concentration | ng/µl or GE/µl [converted] | Second determination of DNA content after nested PCR to evaluate the successful amplification of the region of interest (RoI) within which the targets are located.<br>*The second measurement of DNA concentration is, thus, able to verify how much first amplicon and total DNA template was produced.* |
| Initial target yield (nSP-PCR)[%] | Nested Singleplex PCR Product [GE]/ (($2^n$ - 2n) *x [GE])<br>NOTE: This simplified formula, more precisely the term "($2^n$ - 2n)*x", may be subject to revision, since it must consider stoichiometric aspects of the PCR and its reactants. This equation is a basic model for demonstrating the evaluation concept. | This parameter evaluates the success of the first amplification and will help both in troubleshooting as well as judging whether sufficient template is available to allow for a representative statistic sample and sample size.<br>This can be evaluated by referencing the exponential amplification principle of PCR ($2^n$, where n is the number of cycles).<br>*This correlation can help to assess what the yield of first amplicon relative to template input is.* |

*Flow Cytometry Data Evaluation*

*Evaluation of Initial Target Yield and Statistic Sample Size*

| | | |
|---|---|---|
| Single Beads (SB) (count) | Bead (particle) count in single bead region [gate set by auto-gating function of flow cytometry analysis SW, a vertically streched elliptical gate will be drawn] | Aspiration protocol is defined to count 1 million events *within* the SB gate (not only 1 million events as such!). This guarantees a constant statistic sample size for evaluation of target yield (extended beads).<br>*The single beads count allows assessment of whether the particle number in the aliquot is sufficient for result evaluation.* |
| Single Bead Yield (%) | (# Single beads [within SB auto-gate plot 1] / all beads of plot 1) * 100 [%] | This parameter judges the cleanliness of particle position and particle type detected by the instrument. It serves as another inbuilt QC criterion for the individual measurement of an aliquot.<br>*The single bead yield indicates whether the number of present particles is as expected and whether the position in forward and side scatter is precise enough to allow validation.* |

(continued)

| | | |
|---|---|---|
| SB Population Validation (Position Truth and Orientation) | rCV X and rCV Y of SB population [absolute validation range] AND Orientation of ellipsis: rCVX < rCVY [relative evaluation] AND Position median X and Y [absolute validation range] | This parameter judges the quality of particle position and stray behaviour as such and is in direct correlation to the instrument's QC concerning position and stray behaviour, both characteristics of the position truth principle of flow cytometry that is later used to distinguish mutant from wild type sequence variants of a target region in the biological sample.<br>*The SB population validation is suitable to indicate whether the population phenotype is as expected and whether it indicates basic reliability of particle positions and evaluated sample.* |
| *Gating (Sequential gating) Single beads plot 1 → plot 2.* | | |
| Extended Beads (count) | # EB in EB gate (plot 2) | Number of extended beads must be sufficient to allow the e.g. 0,02% LoD criterion with a sufficient statistic sample size for evaluation. This means that the mutant population still must be evaluable with a sufficient bead number (ideally 100 beads).<br>*Note that LoD claims must be declared per IVD assay individually. The number 0,02% is here only taken as reference for illustration of the concept.* |
| Target Yield (%) | EB# (plot 2)/ all beads (plot 2) [%] | Input parameter for judging plausibility of sample input (template in patient sample) versus yield of extended beads. This parameter will later be used as pre-validation before the actual delta check of the mutant fraction. |

*Gating (Sequential gating) Extended beads plot 2 → plot 3*

*Evaluation of Critical Target Yield and Mutation Status*

| | | |
|---|---|---|
| Wild Type Beads (count) | Bead count in wild type bead target region (auto-gate) | This parameter answers the question whether the number of wild type beads is sufficient to be regarded as valid population size. It is another potential internal control since for the present invention, a negative WT count would be highly unusual. Most human samples have a wild type population, which is mostly significantly bigger in relation to the mutant population. If a rare case of distorted ratio towards an overexpressed mutant population were to occur, we would recommend having an additional medical validation (besides the technical validation covered by data analysis) in place.<br>*This parameter thus allows the assessment whether the WT bead count is representative for human blood sample drawn from body part and with regards to the typical values as reported for respective cancer type.* |
| WT Population Validation (Position Truth + Orientation) | Position median (X and Y), and orientation (rCV X > rCV Y) | Phenotype and position of WT population must match the expected values for the target and be consistent with the mathematical expectation due to the log scaling of plot.<br>*WT Population Validation can indicate whether the wild type population phenotype is as expected to ensure successful amplification of target and performance of instrument and reagents in relation to the analyte.* |

(continued)

| | | |
|---|---|---|
| Mutant Beads (count) | Bead count in mutant bead target region (auto-gate) | The mutant bead count should match the defined minimum value in connection with the extended bead count to ensure a reliable interpretability of population phenotype and mutant fraction.<br>*It assesses whether the MT bead count is high enough to allow mathematical evaluation of the population phenotype.*<br>[Pattern recognition in terms of picture recognition might be possible for smaller bead numbers, but it is not recommendable as standalone.] |
| Mt Population Validation (Position Truth + Orientation) | Position median (X and Y), and orientation (rCVX < rCVY) | Phenotype and position of WT population must match the expected values for the target and be consistent with the mathematical expectation due to the log scaling of plot.<br>*It can, thus, be determined whether the mutant population phenotype is as expected to ensure successful amplification of target and performance of instrument in relation to the analyte.* |
| Critical Target Yield (%) | Beads (Q2/Wt+Q4/Mt) / all beads plot 3 | 1st Level of determining true target yield (and identify off-target beads). Threshold = Cutoff [%]<br>*It can be determined how high the goodness of the fraction value is. Is it sufficient to use it for calculating the absolute fraction?* |
| Mutant Fraction (%) | Mt#/(Mt#+Wt#)*100 [%] | Ratio of DNA in normal physiological state versus pathologic deviation characteristic for a certain cancer type.<br>*This parameter can be used to assess whether the ratio is within the medical validation range for ctDNA from respective blood collection site of body for respective cancer type. This value is compared against the cutoff [mutant fraction as per cent of critical target].* |
| | | |
| *Monitoring and Delta Check* | | |
| Inter-test control: Sample Input versus Target Yield | Sample input up, Target Yield up → ok<br>Sample input up, Target Yield down → irregular<br>Sample input down, target yield down → ok<br>Sample input down, target yield up → irregular | This plausibility verification for the trend analysis considers both the DNA content of the sample (= preconditions for assay performance as well as inspection of patient sample quality control) and the target yield in relation to the template input. It accounts for the fact that automation will usually pipet a standard/fixed volume of template input into the PCRs. Thus, variation in template input concentration must be considered.<br>*If sample DNA concentration trend (compared to previous test run) correlates with target yield this is assumed as regular performance of the assay in relation to sample quality. This is required to avoid any errors resulting from variations caused by sample quality and/or assay performance.* |
| Mutant Fraction Delta Check (relative fraction) | Delta between T1 and T2 to Tn and Tm Mutant Fraction values (validation range to be defined per assay) | The concept of delta check is in line to other diagnostic monitoring methods and will ensure that unusual changes in mutation status and fraction are indicated to prevent false conclusions on the effect of a treatment. Usually, a measurement might be repeated if the test result does not fit in with the other tests and the (expected) trend in patient's status development. The fraction delta may be set assay or test specific, depending on available data. This remains subject to medical validation.<br>*Is the mutant fraction value within the expected delta as compared to the previous measurement?* |

*Other parameters*

The selection and establishment of these three parameter definitions can be established per application (assay) and in accordance with the data available for a particular cancer type. Therefore, the calculation example below focuses on the generic parameters which can be applied to all applications of the invention equivalently.

| | | |
|---|---|---|
| Deviation from Expected EB Count | EB count / $(2^n - 2n)*x$; x = Template DNA [GE]; Note that this equation can be modified to include the evaluation against the maximum number of coupling sites per bead to estimate whether a distortion in wild type/ mutant ratio has occurred due to experimental variations. | Theoretically, one template molecule would bind to one bead. The PCR is optimized in such way as to yield the expected number of beads (corresponding to template input (x)), but at least in a correlative (ratio) manner to ensure a representative mutant fraction calculation (risk mitigation in case more than 1 template DNA strands bind to a bead). This formula (the value 'x') depends on the reaction product of the nested PCR and the respective template input (as calculated via pipetted template volume and DNA concentration of nested PCR product).<br>*It can be assessed whether the suspension PCR was successful in providing a representative target yield.* |
| Absolute Mutant Fraction | DNA concentration Sample * Mutant Fraction | *It can be determined what the absolute concentration of ct DNA within cfDNA is.*<br>*Note that this value, since it relies on the DNA concentration of the sample as a whole, may also be subject to variation due to sample quality. Therefore, the delta check is devided into two levels - the relative and the absolute mutant fraction. This must be considered to avoid distorted trend curves for monitoring.* |
| Mutant Fraction Delta Check (absolute fraction) | Delta between T1 and T2 to Tn and Tm Mutant Fraction values (validation range to be defined per assay) | This delta check refers to the absolute quantification of ct DNA within the cf DNA.<br>*Is the mutant fraction value within the expected delta as compared to the previous measurement?*<br>*Note that the absolute mutant fraction is directly dependent on DNA concentration of sample. Possible effects are discussed in a later example.* |

***Further aspects of the present invention***

The above-described method of the invention can be used in an *in vitro* diagnostic method. Thus, in a second aspect, the invention pertains to an *in vitro* method for diagnosis of a disease associated with a mutation in a nucleic acid sequence, comprising the steps of

(A) extracting nucleic acids from a sample of a patient to form an analytic sample;

(B) detecting and/or quantifying the nucleic acid sequence and, optionally, a variant of the nucleic acid sequence in the analytic sample, wherein detection and/or quantification is according to the method of the invention, and

wherein detection and/or quantification of the variant is carried out successively or simultaneously with regard to the detection and/or quantification of the nucleic acid sequence.
In a third aspect, the invention relates to a device for detection and/or quantification of at least one nucleic acid sequence in a sample comprising

(i) a measurement unit for the measurement of nucleic acid concentration;
(ii) a thermal cycler module for polymerase chain reaction and hybridization reaction;
(iii) a holder unit optionally moveable at least in z-direction and/or optionally capable of shaking an entity held by the holder unit;
(iv) at least two electromagnetic units; and
(v) a PCR preparation unit;
(vi) at least one stack for microtiter plates; and
(vii) at least one flow cytometry unit.

The components (i) to (vii) are operably connected and arranged within the device in a manner that enables the device to carry out the method of the invention in an automated or at least semi-automated manner. Preferably, the device is able to carry out all method steps automatically and without human intervention. For this purpose, the device

can further comprise a control unit, e.g. a computer, which coordinates the different components and calculates the results of the analysis in accordance with the principles outlined elsewhere herein.

The shaking in the holder unit can be achieved by vibrating the unit or a unit comprised therein and, thus, shaking the entity held by the holder unit. The entity may, for example, be a microtiter plate or the like. Shaking may occur gently and with a high frequency ("vortexing").

In a fourth aspect, the invention pertains to a system for detection and/or quantification of at least one nucleic acid sequence in a sample, comprising

(I) a device according to the present invention; and

(II) a second device for isolating and quantifying nucleic acids from a biological sample; wherein the second device comprises

(IIa) a nucleic acid extraction unit;

(IIb) a measurement unit for the measurement of nucleic acid concentration;

(IIc) at least one stack for microtiter plates;

(IId) a PCR preparation unit;

(IIe) optionally, a pre-analytic sample sorting unit;

(IIf) at least one pipetting device for transfer of sample and other liquids from sample tubes to reaction vials and/or plates; and

(IIg) at least one pipetting device for reagent transfer from reagent reservoirs to reaction vials and/or plates.

The components (IIa) to (IIg) are operably connected and arranged within the second device in a manner that enables the second device to isolate and quantify nucleic acids from a biological sample in an automated or at least semi-automated manner. Preferably, the second device is able to isolate and quantify nucleic acids from a biological sample automatically and without human intervention. For this purpose, the device can further comprise a control unit, e.g. a computer, which coordinates the different components.

The pre-analytic sample sorting unit (Iid) comprises preferably at least one rack holder unit and at least one device (e.g. robot arm) capable of grabbing, releasing, decapping, and recapping of sample tubes and/or microtiterplates.

Pipetting devices which can be used in the devices according to the invention are known in the art and include *inter alia* pipetting arms and the like.

The second device will be able to provide a purified nucleic acid sample which can readily be used as an input sample for the first device according to the invention. Thus, the system according to the invention can be used for a thorough and semi-automated quantification and detection of a nucleic acid in a biological sample, such as a plasma sample.

## Brief description of the Figures

**Fig. 1** schematically shows the sequence of steps of one embodiment of the method of the invention, wherein 1 A schematically shows method steps (a) to (d) and 1 B schematically shows method steps (f) to (g);

**Fig. 2** schematically shows three different graphical displays of flow cytometric results obtained in the method of the invention; (SSC: Side Scatter (capable to detect all particle sizes and ranges [nm] within range of cytometer specification / configuration); FSC: Forward Scatter (capable to detect all particle sizes and wavelength ranges [nm] within cytometer specification / configuration); Channel A: Fluorescence channel capable of detecting both fluorescent probe signals (WT and MT) [range a-f nanometer]; Channel B: Fluorescence channel capable of detecting MT fluorescent probe signals [range b-c nanometer]; Channel C: Fluorescence channel capable of detecting WT fluorescent probe signals [range d-e nanometer]; Range a-f encompasses ranges b-c, d-e; 2D plots represent overlays of respective data collections for defined channels for an individual sample measurement (log scale); Positions of gates may vary per analyte/target. Proportions distorted for illustration purposes; may vary for real gate sizes and data sets);

**Fig. 3** schematically shows the same graphical display of flow cytometric results of plot 2 shown in Fig. 2, i.e. the differentiation of extended and non-extended beads via mapping/overlay of respective fluorescence channels, obtained in the method of the invention for three exemplary diagnostic results (A: Mutation positive; B:Mutation negative (wild type); C: No template);

**Fig. 4** schematically shows plot 3 shown in Fig. 2, i.e. the separation of target variants, wild type versus mutant, via mapping/overlay of respective fluorescence channels, graphically displayed for the three exemplary diagnostic results (A: Mutation positive; B: No template; C: Mutation negative (wild type); and

**Fig. 5** illustrates the distorted relation between relative and absolute mutant fraction when not normalized or plotted against DNA content.

**Examples**

The above disclosure generally describes the present invention. A more complete understanding can be obtained by reference to the following specific examples which are provided herein for the purpose of illustrating the invention only, and are not intended to limit the scope of the invention.

**Example 1: DNA Extraction**

DNA is extracted from a biological sample by a method appropriate for the sample type (tissue or plasma). For plasma samples, extraction is done via centrifugation-free and bead-free extraction method (e.g. by Pressure and Immiscibility-Based EXtraction (PIBEX) described in Lee et al., 2018, Scientific Reports, 8, Article number: 5467). The eluate is used as sample input (template) for the TESQ method described in Example 2.

**Example 2: TESQ (Target Enrichment - Suspension and Quantification)**

State of the art products which are suitable for qualitative determination of the mutation status in *in* vitro diagnostics include e.g. the Biocartis Idylla ctKRAS Test (Biocartis, 2018) covering 21 mutations in codons 12, 13, 59, 61, 117 and 146 of the KRAS gene and the therascreen KRAS RGQ PCR Kit covering seven mutation-specific reactions in codons 12 and 13 of exon 2 of the KRAS oncogene, and a wild type control in exon 4 (Qiagen, 2012). The mutations covered by these kits are listed in the below table.

| **Assay (Product)** | **ctKRAS (CE IVD) (Biocartis)** | **Therascreen KRAS (CE IVD) (Qiagen)** |
|---|---|---|
| Mutations covered | Codon 12 (exon 2)<br>G12C (c.34G>T)<br>G12R (c.34G>C)<br>G12S (c.34G>A)<br>G12A (c.35G>C)<br>G12D (c.35G>A)<br>G12V (c.35G>T)<br>Codon 13 (exon 2)<br>G13D (c.38G>A)<br>Codon 59 (exon 3)<br>A59E (c.176C>A)<br>A59G (c.176C>G)<br>A59T (c.175G>A)<br>Codon 61 (exon 3)<br>Q61K (c.181C>A; c.180_181 delinsAA)<br>Q61L (c.182A>T)<br>Q61R (c.182A>G)<br>Q61H (c.183A>C; c.183A>T)<br>Codon 117 (exon 4)<br>K117N (c.351A>C; c.351A>T)<br>Codon 146 (exon 4)<br>A146P (c.436G>C)<br>A146T (c.436G>A)<br>A146V (c.437C>T) | Codon 12 (exon 2)<br>GLY12ALA (G12A) GGT>GCT<br>GLY12ASP (G12D) GGT>GAT<br>GLY12ARG (G12R) GGT>CGT<br><br>GLY12CYS (G12C) GGT>TGT<br>GLY12SER (G12S) GGT>AGT<br>GLY12VAL (G12V) GGT>GTT<br>Codon 13 (exon 2)<br>GLY13ASP (G13D) GGC>GAC |

The results that can be obtained with the aforementioned state of the art tests are qualitative. A quantitative assessment of the number of mutant genes in relation to the wild type sequences is not possible with these tests.

To illustrate the concept of the method of the invention, a hypothetical panel is evaluated for comparison. This panel includes the following individual mutation tests and controls:

| **Mutation Panel** | **Test** | **External Controls** |
|---|---|---|
| G12C (c.34G>T) | *KR2 Cdn12* G12C | *PC (MT)*<br>*NC (WT)* |
| G12R (c.34G>C) | *KR2 Cdn12* G12R | *PC (MT)*<br>*NC (WT)* |
| G12S (c.34G>A) | *KR2 Cdn12* G12S | *PC (MT)*<br>*NC (WT)* |
| G12A (c.35G>C) | *KR2 Cdn12* G12A | *PC (MT)*<br>*NC (WT)* |
| G12D (c.35G>A) | *KR2 Cdn12* G12D | *PC (MT)*<br>*NC (WT)* |
| G12V (c.35G>T) | *KR2 Cdn12* G12V | *PC (MT)*<br>*NC (WT)* |
| G13D (c.38G>A) | *KR2 Cdn13* G13D | *PC (MT)*<br>*NC (WT)* |
| Panel | | *NTC (No template)* |

The results that can be obtained with the method of the invention allow a quantitative determination of the DNA concentration and relative and absolute MAF (mutant allele fraction). The cutoff for the exemplary panel is 0,02% mutant fraction, and applicable to all tests. Individual test result evaluation is conducted as described elsewhere herein in detail.

*a. Quantification of DNA input concentration*

DNA input concentration is determined via microvolume spectrometry.

*b. Target enrichment by Nested/Singleplex PCR (Exon-specific)*

Using a nested, singleplex PCR with exon-specific primers, the region of interest (RoI) is amplified. The amplification intends to cover all targets within one exon, permitting a specific and high target yield. Suitable nested, singleplex PCR methods are known in the art and described e.g. by Elnifro (2000, Clin Microbiol Rev, 559-570), DeBiasi (1999, Arch Neurol, 56, 1215-1219), Shin (2012, Anticancer Res,32: 163-168), Wang (2015, Asian Pac J Cancer Prev, 16 (7), 3003-3007) and Reinard (2018, Molekularbiologische Methoden 2.0. 2nd ed. Stuttgart: utb, 2018:, 85 f.)

The DNA polymerase used is a thermostabile enzyme variant. PCR reagents permit storage at room temperature and have an open-bottle stability of up to 60 days. Room temperature (RT) is hereby defined as 20°C +/- 5°C, i.e. a range of 15-25°C. A temperature of 21°C is preferred. The enzyme in the reagent mixture is stabilized by enzyme conservation, and its activity for PCR is induced by trespassing a temperature threshold, allowing it to enter the preferable reaction with thermodynamically favourable reactants - in this case the dNTPs and the single-stranded template DNA (sample input). Enzyme conservation during storage is achieved by masking of the binding site and/or lyophylization (also refer to Bedu-Addo, 2004, Pharmaceut Technol, Lyophilization, 10-18).

*i. Quantification of target yield (DNA content)*

The DNA target yield from the described target enrichment PCR is quantified using microvolume spectrometry to validate that target enrichment was successful and to quantify the yield. The number of template molecules originally obtained from the biological sample and DNA template yield is determined (= calculated using analysis Software (SW); a customized computer program) against the DNA input concentration of step a).

This input concentration of first amplicons subjected to suspension PCR in step c (yielding second amplicons) will later be used to judge the success of the suspension PCR output and at the same time, to judge the goodness of

mutant fraction (relative).

If a bead only had one binding site, it were expected to bind one single DNA (template) strand in the suspension PCR. However, depending on the surface, beads have several binding sites (depending on surface-to-volume ratio). Since the number of template input is known (can be determined based on the concentration measured in the nested PCR reaction product and by multiplication with the applied template input volume to the suspension PCR, and by conversion to GE / copy number), deviations from expected yield can be determined. Since the number of binding sites is statistically equivalent for a constant bead diameter, the possible additional binding events can be determined. In sum, the ratio of template input (single copies) to beads must be plausible. In addition, an internal and/or external control for validating the binding events with beads can be implemented.

*c. Target-specific suspension PCR*

This PCR uses a set of light-weight magnetic beads (1:1 ratio in reagent) to which either wild type - or mutant variant-specific primers have been coupled. Suitable beads are known in the art and include *inter alia* the "Streptavidin-coupled Dynabeads" (magnetic) beads from Thermo Fisher Scientific. The beads have a defined surface-to-volume-ratio, statistically ensuring the same number of possible primer-binding sites on each bead. Primers can be coupled to the beads as described in e.g. Novex (2012, see above).

The first amplicons gained in the target enrichment PCR described above are used as templates in this suspension PCR.

The primer-coupled beads and templates are resuspended in an aqueous, non-polar buffer to provide a homogenous distribution within the buffer. PCR cycles are optimized for optimal yield of second amplicons. Through repeated PCR cycles, the beads' binding sites are complemented with second amplicon DNA of wild type or mutant sequence respectively.

The resulting solution comprises "second" amplicons bound to beads.

*d. Hybridization with target-specific primers and fluorescence-labelled probes*

Denatured DNA single strands of second amplicons bound to the beads are hybridized with fluorescently labeled probes that are specific for either the mutant or wild type sequence. The hybridization product is washed to remove residual reagents and in particular the probes that were not bound to the beads. Subsequently, the beads are resuspended in buffer solution (e.g. PBS buffer).

*e. Flow Cytometry*

The labelled template-bound beads are then analyzed using flow cytometry. Suitable flow cytometric methods are known in the art and described e.g. in Witt (2017, Eng Life Sci, 17, 953-958), Garcia (2018, Oncotarget, 9(30), 21122-21131), Shapiro (Practical Flow Cytometry. 4th ed. NewYork: Wiley-Liss, 2003: pp. 231-236.Print.), Diehl (2006, BEAMing: single-molecule PCR on microparticles in water-in-oil emulsions, Nature Methods, 3: 551-559). Flow cytometry allows separating the mutant and wild type variants by detection of the fluorescent signals. The beads are separated into homogenous wild type and mutant clusters (populations) of the particular target.

*f. Data and result analysis*

Using a sequential gating strategy as described in Shapiro (2003) and FCS Express Software User Manual (De Novo Software, FCS EXPRESS 6 RUO EDITION USERS MANUAL. [Software User Manual], 2016, 2019) to analyze the flow cytometry data allows the identification of data subsets and the target separation and subsequently the calculation of the yield of each variant analyzed. As also described in De Novo (Software, FCS EXPRESS 6 RUO EDITION USERS MANUAL. [Software User Manual], 2016, 2019), Shapiro (2003), Diehl (2008, Nat Med, 14(9):985-90) and Verschoor (2015, Front Immunol, 6:380), the sequential (or hierarchical) gating strategy identifies individual particles (here: single beads) and their data subsets (here: extended beads versus non-extended beads). "Extended" beads are magnetic beads to which template DNA strands and their amplicons have bound and which have been fluorescently labeled with the respective / corresponding mutant or wild type - specific probe. Finally, the extended beads are subdivided into beads attached to second amplicons of the mutant sequence and beads attached to second amplicons of the wild type sequence.

The differentiation between these two types of beads is made based on the principle of position truth for a distinct molecule, which herein is represented by the equivalent molecule structure within the particular wild type variant and mutant variant of a specific target sequence within a gene region to be analyzed. The sum of all analyzed mutant and all wild type-labelled beads each form a separate population, being located in a different region that is repeatably

checked/identified by a target gate.

Potentially occurring unspecific reaction products are identified within off-target regions and by judging the stray behaviour and orientation or shape of a population which results from the interaction of the analyte with the cytometer, as well as the selected resolution and scaling in analysis plots.

Plausibility and confidence of the result values is validated by comparison with the template and sample input concentrations, the in-process-control of the first amplicon yield and the expected total extended bead yield against tolerable error to ensure the delivery of a reliable mutant fraction value, both relative and absolute. Suitable (statistical) approaches are described e.g. in Shapiro (2003) for flow cytometry applications and FDA guidances for PCR-based diagnostic applications (2011, 2014).

The table below describes an example of distorted ratios of DNA concentrations versus absolute and relative mutant fractions. Taback et al (2004, Ann NY Acad Sci, 1022:17-24; p. 19) described high cfDNA concentrations of 259 ng/ml plasma in stage IV melanoma patients, which corresponds to 259 pg/μl. In a haploid genome, this could be converted into 78 GE/μl, meaning 78 000 GE/ml plasma (considering 1 GE/μl in a haploid genome corresponds to 3,3 pg/μl). The table below assumes exemplary DNA concentrations at the lower limit of required input concentration for the assay.

| Time point | Sample ID | Mutant Fraction relative [%] | Total cfDNA [GE, haploid] | MT Fraction absolute [GE] |
|---|---|---|---|---|
| T1 | S-PT1 | 6,8 | 10500 | 111,8 |
| T2 | S-PT2 | 1,6 | 9300 | 94,6 |
| T3 | S-PT3 | 0,07 | 8250 | 83,2 |
| T4 | S-PT4 | 0,08 | 8200 | 82,1 |
| T5 | S-PT5 | 0,07 | 5000 | 50,1 |

Fig. 5 illustrates the distorted relation between relative and absolute mutant fraction when not normalized or plotted against DNA content. Depending on which parameters are of interest for monitoring, the data can be provided in three ways:

1) the cell free DNA content and the mutant allele fraction (relative);
2) mutant allele fraction; and
3) normalized fraction value which is adjusted considering the different DNA input concentrations.

To avoid any false assumptions due to artificial conversion of the data, it is preferred to use the non-normalized and integrated presentation of both MAF and DNA concentration parameters.

A hypothetical scenario for the KR2 Cdn12 and 13 panel described above in comparison to the Qiagen and Biocartis products, monitoring is provided below. In two results a delta check warning is triggered. In the test *KR2 Cdn12* G12S, the delta check warning is triggered due to a significant change in the MAF value, in the second case, *KR2 Cdn12* G12D, the user is notified because the mutation status has changed from negative to positive. The delta value must be determined per mutation test.

| Test | Result T1 [MAF %] | Controls | Status | Result T2 [MAF %] | Controls | Status | Delta Check |
|---|---|---|---|---|---|---|---|
| *KR2 Cdn12 G12C* | 0,069 | Valid | POS | 0,050 | Valid | POS | Ok |
| *KR2 Cdn12* G12R | 1,250 | Valid | POS | 0,980 | Valid | POS | Ok |
| *KR2 Cdn12* G12S | 4,330 | Valid | POS | 0,015 | Valid | POS | Delta Check! |
| *KR2 Cdn12* G12A | 0,300 | Valid | POS | 0,300 | PC invalid | INV | Ok |
| *KR2 Cdn12* G12D | 0,005 | Valid | NEG | 0,060 | Valid | POS | Mutation Status! |

(continued)

| Test | Result T1 [MAF %] | Controls | Status | Result T2 [MAF %] | Controls | Status | Delta Check |
|---|---|---|---|---|---|---|---|
| *KR2 Cdn12* G12V | 0,800 | Valid | POS | 0,650 | Valid | POS | Ok |
| KR2 Cdn13 G13D | 0,004 | Valid | NEG | 0,008 | Valid | NEG | Ok |

Legend Result Values: POS = mutation detected/on or above, NEG = no mutation detected/below cutoff, INV = invalid; T = time point, MAF = Mutant Allel Fraction; Delta check for MAF triggered for $\Delta > 2\%$.Note that this is a hypothetical delta value for demonstration of principle. The delta value for triggering mutant fraction-related delta must be determined for each individual assay, or even test based on clinical evidence.

**Example 3: Mutation-positive, valid run (a hypothetical scenario)**

The calculation example below uses a set of given hypothetical values considering experimentally caused deviation:

- DNA Input concentration of biological sample: 6500 GE (haploid Genome)
- Minimum requirement DNA input: 5000 GE (haploid Genome; acc. Garcia et al, 2018, "Oncotarget". Vol. 9 (No. 30): p. 21125-21126)
- nSP PCR: Total of 30 cycles
- Hypothetical nSP PCR product concentration [GE]: $6{,}700 \cdot 10^{12}$
- Total Bead Count: 1009870
- Single Beads: 1000000 (1 Million)
- Extended Beads: 203006
    - Wild Type Beads: 202844
    - Mutant Beads: 128
    - Noise (beads) Plot 3 off-target regions: 34
- Suspension PCR: 20 cycles (according to Witt, 2017, Eng Life Sci, 17, 953-958)

Template input volume to suspension PCR will be determined application/assay-specific and considering the required/optimum DNA template input. To simulate experimental variation as it can occur in sample measurements (resulting from variation in biological sample quality), we assume a hypothetical extended bead yield.

| Test Parameter | Unit / Formula / Function | Result Value /Validation Status / Interpretation |
|---|---|---|
| DNA concentration (Template input) | GE<br>Target Value: ≥ 5000 GE | 6500 GE<br>→ in spec |
| nSP PCR Product (DNA) Concentration | GE | $6{,}700 \cdot 10^{12}$ |
| Initial target yield (nSP-PCR) | (nSP PCR Product [GE]/ $((2^n - 2n) \cdot x)$: $(6{,}700 \cdot 10^{12} / 6{,}979 \cdot 10^{12})$ Target range: ≥ 0,95 | 0,96<br>→ in spec |
| Single Beads (count) | Bead (particle) count in single bead region [gate set by auto-gating function of flow cytometry analysis SW, a vertical elliptical gate will be drawn] | 1 Mio<br>→ in spec |
| Single Bead Yield (%) | (# Single beads [within SB auto-gate plot 1] / all beads of plot 1) * 100 [%]<br>Target range: ≥ 95%<br>This range may be subject to change. | 1 Mio/1009870 = 0,990 *100% = 99,0%<br>→ in spec |

(continued)

| Test Parameter | Unit / Formula / Function | Result Value /Validation Status / Interpretation |
|---|---|---|
| SB Population Validation (Position Truth and Orientation) | rCV X and rCV Y of SB population [absolute validation range; approximation to 1:2 ratio for x/y orientation] AND Orientation of ellipsis: rCVX < rCVY [relative evaluation] AND Position median X and Y [absolute validation range, target-specific] | rCV X/rCV Y = 22,5 / 38,9 → in spec<br>position median X: in spec<br>position median Y: in spec |
| Extended Beads (count) | # EB in EB gate (plot 2) | 203006 |
| Target Yield (%) | EB# (plot 2)/ all beads (plot 2) [%] | (203006/1000000)*100% = 20,3% |
| Wild Type Beads (count) | Bead count in wild type bead target region (auto-gate) | 202844 |
| WT Population Validation (Position Truth + Orientation) | Position median (X and Y), and orientation (rCV X > rCV Y) | rCV X/rCV Y = 43,5 / 25,2 → in spec<br>position median X: in spec<br>position median Y: in spec |
| Mutant Beads (count) | Bead count in mutant bead target region (auto-gate) | 128 |
| Mt Population Validation (Position Truth + Orientation) | Position median (X and Y), and orientation (rCV X < rCV Y) | rCV X/rCV Y = 22,5 / 38,9 → in spec<br>position median X: in spec<br>position median Y: in spec |
| Critical Target Yield (%) | (Q2/Wt# + Q4/Mt#) / (beads plot 3) * 100 [%] | (202844+128) / 203006 = 0,99<br>*100% = 99% |
| Mutant Fraction (%) [relative fraction] | Mt#/(Mt#+Wt#)*100 [%] | (128/(202844+128))*100 [%] = **0,06%** |
| **Delta check** | | |
| Inter-test control: Sample Input versus Target Yield | Sample input up, Target Yield up → ok<br>Sample input up, Target Yield down → irregular<br>Sample input down, target yield down → ok<br>Sample input down, target yield up → irregular | Not applicable (example assumes as first measurement) |
| Mutant Fraction Delta Check relative and absolute | Delta between T1 and T2 to Tn and Tm Mutant Fraction values (validation range to be defined per assay) | Not applicable (example assumes as first measurement) |

## Claims

**1.** A method for detection and/or quantification of at least one nucleic acid sequence in a sample, comprising the steps of:

(a) optionally measuring the nucleic acid concentration in the sample;
(b) amplifying the at least one nucleic acid sequence by nested polymerase chain reaction using nucleic acids comprised in the sample as template to form a set of first amplicons comprised in a first reaction mixture;
(c) optionally measuring the nucleic acid concentration in the first reaction mixture;
(d) amplifying the at least one nucleic acid sequence by polymerase chain reaction using the set of first amplicons as template and using nucleotide primers which are specific for the at least one nucleic acid sequence and which are coupled to magnetic beads to form a set of bead-coupled second amplicons comprised in a second reaction mixture;

(e) optionally isolating the magnetic beads from the second reaction mixture;
(f) combining the magnetic beads from step (d) or (e) with a labeled probe, wherein the probe binds specifically to the nucleic acid sequence;
(g) detecting and/or quantifying magnetic beads labeled with the probe; and
(h) determining the presence and/or the amount of the at least one nucleic acid sequence in the sample based on the detection and/or quantification in step (g).

**2.** The method of claim 1, wherein at least two nucleic acid sequences are detected and/or quantified.

**3.** The method according to any of the preceding claims, wherein the detection and/or quantification in step g) is carried out with flow cytometry.

**4.** The method according to any of the preceding claims, wherein the measurement of nucleic acid concentration in step (a) and/or (c) is carried out with microvolume spectrometry.

**5.** The method according to any of the preceding claims, wherein the nested polymerase chain reaction of step b) is a nested singleplex polymerase chain reaction.

**6.** The method according to any of the preceding claims, wherein the polymerase chain reaction of step d) is a multiplex polymerase chain reaction.

**7.** The method according to any of the preceding claims, wherein the polymerase chain reaction of step d) is carried out in a suspension.

**8.** The method according to any of the preceding claims, wherein the beads have

(I) a defined diameter, preferably a diameter from 0,1 to 2 $\mu$m;
(II) a defined number of coupling sites on their surface;
(III) optionally, magnetic properties;
(V) a size distribution with CV $\leq$ 5 % (in diameter); and
(VI) an iron content of $\leq$ 30 %.

**9.** The method according to any of the preceding claims, wherein the probe is a fluorescently labeled probe.

**10.** An *in vitro* method for diagnosis of a disease associated with a mutation in a nucleic acid sequence, comprising the steps of

(A) extracting nucleic acids from a sample obtained from a patient to form an analytic sample;
(B) detecting and/or quantifying the nucleic acid sequence and, optionally, a variant of the nucleic acid sequence in the analytic sample, wherein detection and/or quantification is according to the method of any of claims 1 to 9, and

wherein detection and/or quantification of the variant is carried out successively or simultaneously with regard to the detection and/or quantification of the nucleic acid sequence.

**11.** A device for detection and/or quantification of at least one nucleic acid sequence in a sample comprising

(i) a measurement unit for the measurement of nucleic acid concentration;
(ii) a thermal cycler module for polymerase chain reaction and hybridization reaction;
(iii) a holder unit, optionally, moveable at least in z-direction and/or optionally capable of shaking an entity held by the holder unit;
(iv) at least two electromagnetic units;
(v) a PCR preparation unit;
(vi) at least one stack for microtiter plates; and
(vii) at least one flow cytometry unit.

**12.** A system for detection and/or quantification of at least one nucleic acid sequence in a sample, comprising

(I) a device according to claim 11; and
(II) a second device for isolating and quantifying nucleic acids from a biological sample; wherein the second device comprises

(IIa) a nucleic acid extraction unit;
(IIb) a measurement unit for the measurement of nucleic acid concentration;
(IIc) at least one stack for microtiter plates;
(IId) a PCR preparation unit;
(IId) optionally, a pre-analytic sample sorting unit;
(IIe) at least one pipetting device for transfer of sample and other liquids from sample tubes to reaction vials and/or plates; and
(IIf) at least one pipetting device for reagent transfer from reagent eservoirs to reaction vials and/or plates.

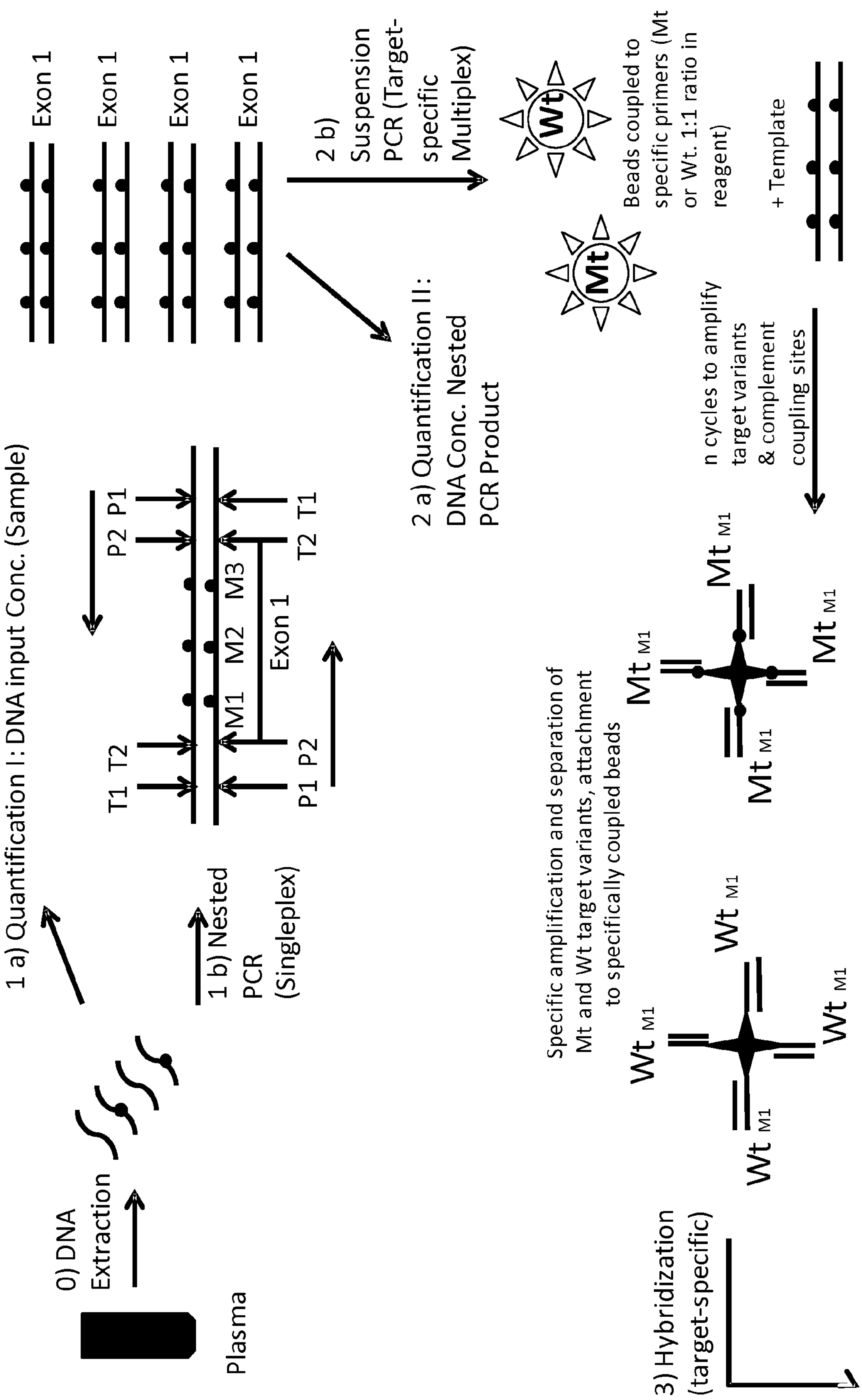
Plasma
0) DNA Extraction
1 a) Quantification I: DNA input Conc. (Sample)
1 b) Nested PCR (Singleplex)
T1 T2
P2 P1
M1 M2 M3
Exon 1
P1 P2
T2 T1
2 a) Quantification II: DNA Conc. Nested PCR Product
Exon 1
Exon 1
Exon 1
Exon 1
2 b) Suspension PCR (Target-specific Multiplex)
Mt
Wt
Beads coupled to specific primers (Mt or Wt. 1:1 ratio in reagent)
+ Template
n cycles to amplify target variants & complement coupling sites
Specific amplification and separation of Mt and Wt target variants, attachment to specifically coupled beads
Mt M1
Mt M1
Mt M1
Mt M1
Wt M1
Wt M1
Wt M1
Wt M1
3) Hybridization (target-specific)

Fig. 1A

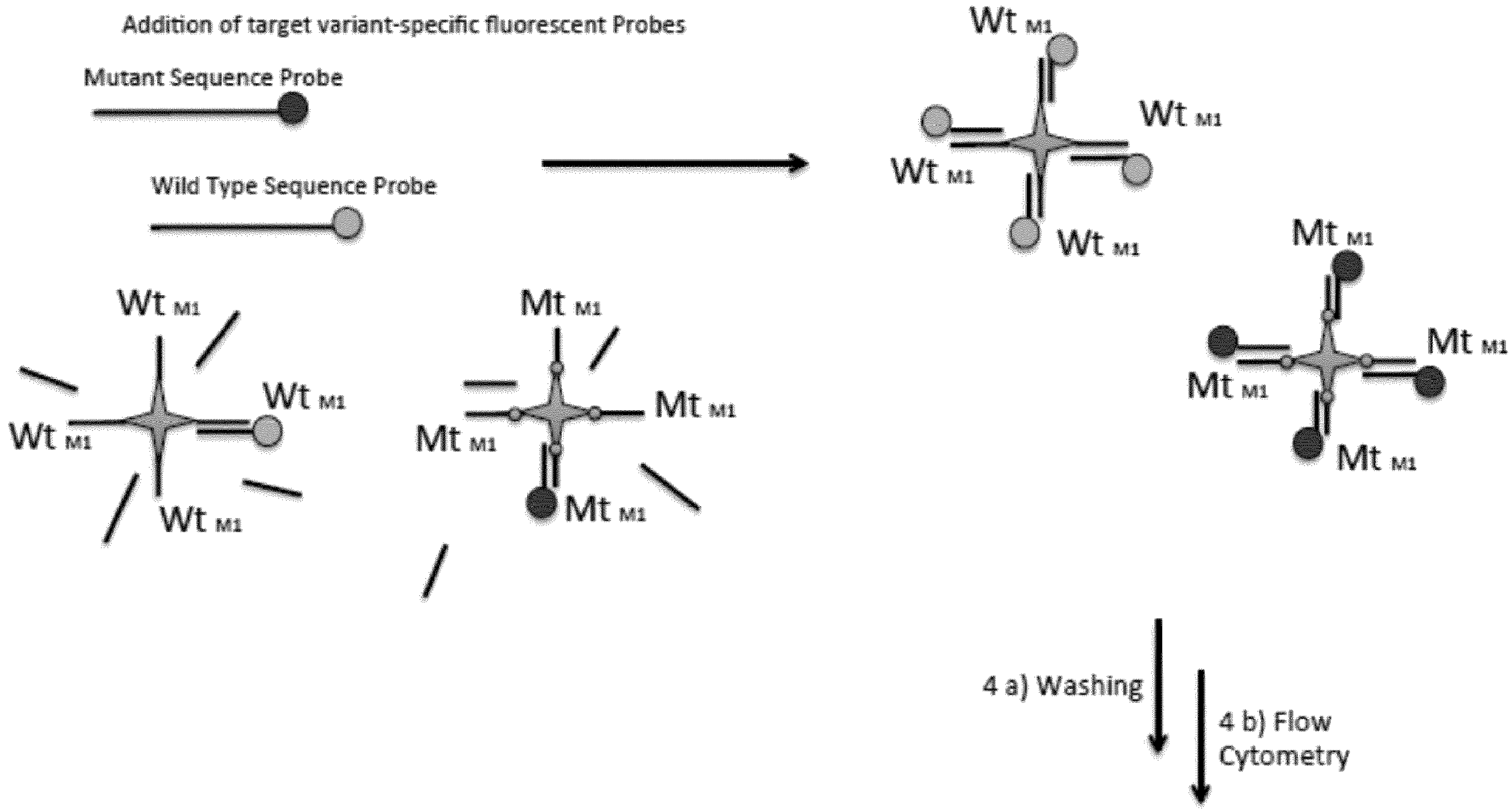
Addition of target variant-specific fluorescent Probes
Mutant Sequence Probe
Wild Type Sequence Probe
Wt M1
Mt M1
4 a) Washing
4 b) Flow Cytometry

Fig. 1B

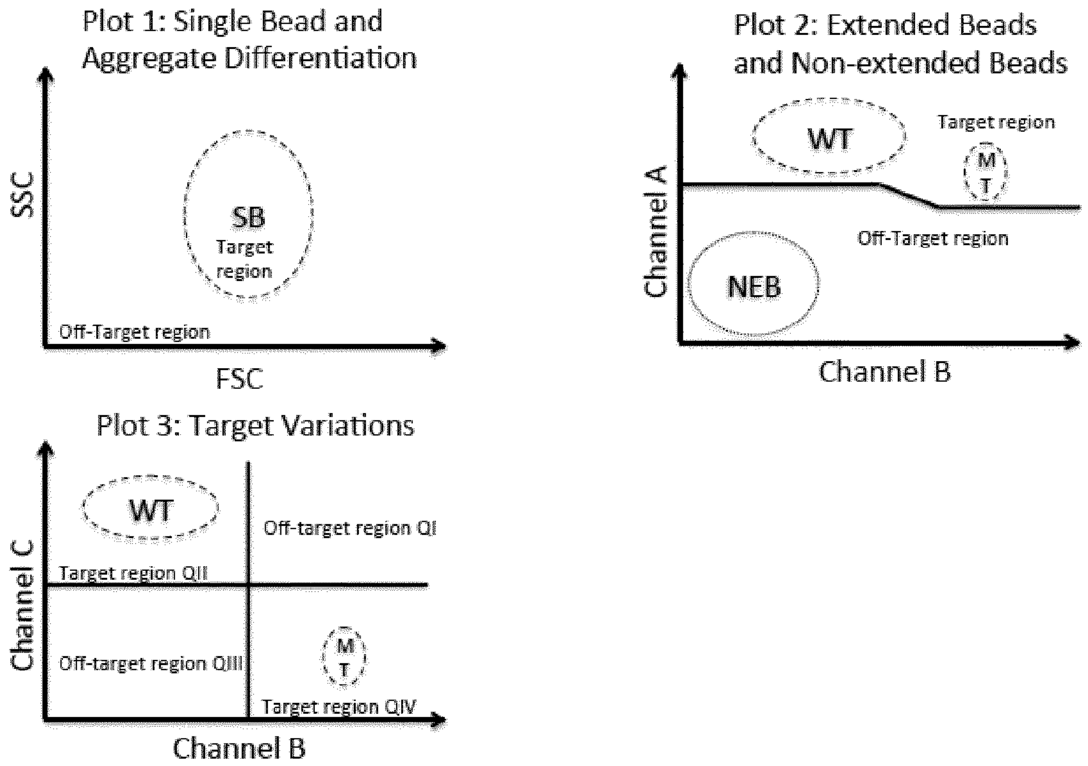
Plot 1: Single Bead and Aggregate Differentiation
SSC
SB
Target region
Off-Target region
FSC
Plot 2: Extended Beads and Non-extended Beads
Channel A
WT
Target region
MT
Off-Target region
NEB
Channel B
Plot 3: Target Variations
Channel C
WT
Off-target region QI
Target region QII
Off-target region QIII
MT
Target region QIV
Channel B

Fig. 2

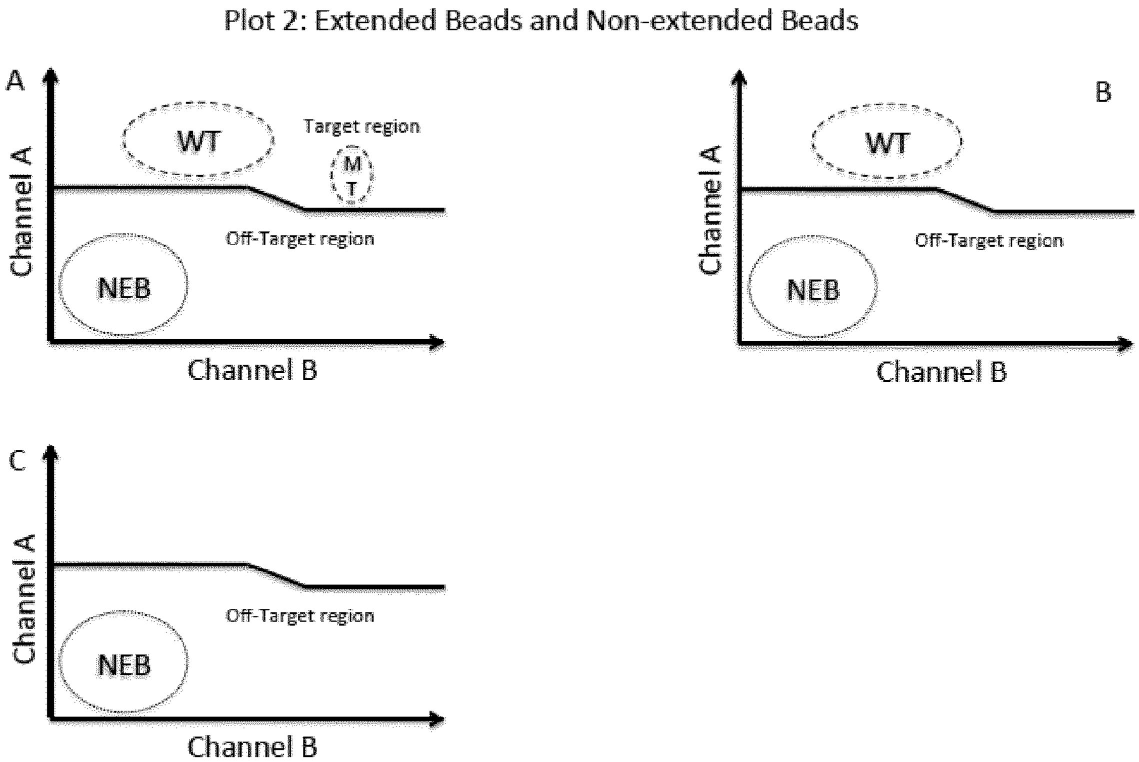
Plot 2: Extended Beads and Non-extended Beads
A
Channel A
WT
Target region
MT
Off-Target region
NEB
Channel B
B
Channel A
WT
Off-Target region
NEB
Channel B
C
Channel A
Off-Target region
NEB
Channel B

Fig. 3

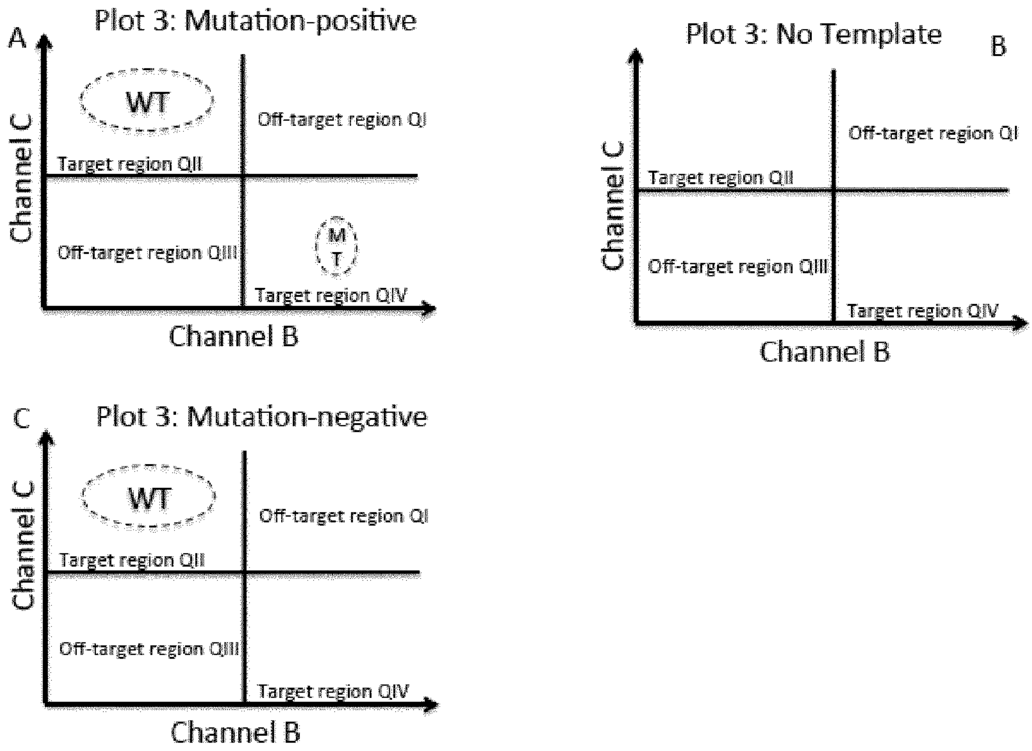
A
Plot 3: Mutation-positive
WT
Off-target region QI
Target region QII
Off-target region QIII
MT
Target region QIV
Channel C
Channel B
B
Plot 3: No Template
Off-target region QI
Target region QII
Off-target region QIII
Target region QIV
Channel C
Channel B
C
Plot 3: Mutation-negative
WT
Off-target region QI
Target region QII
Off-target region QIII
Target region QIV
Channel C
Channel B

**Fig. 4**

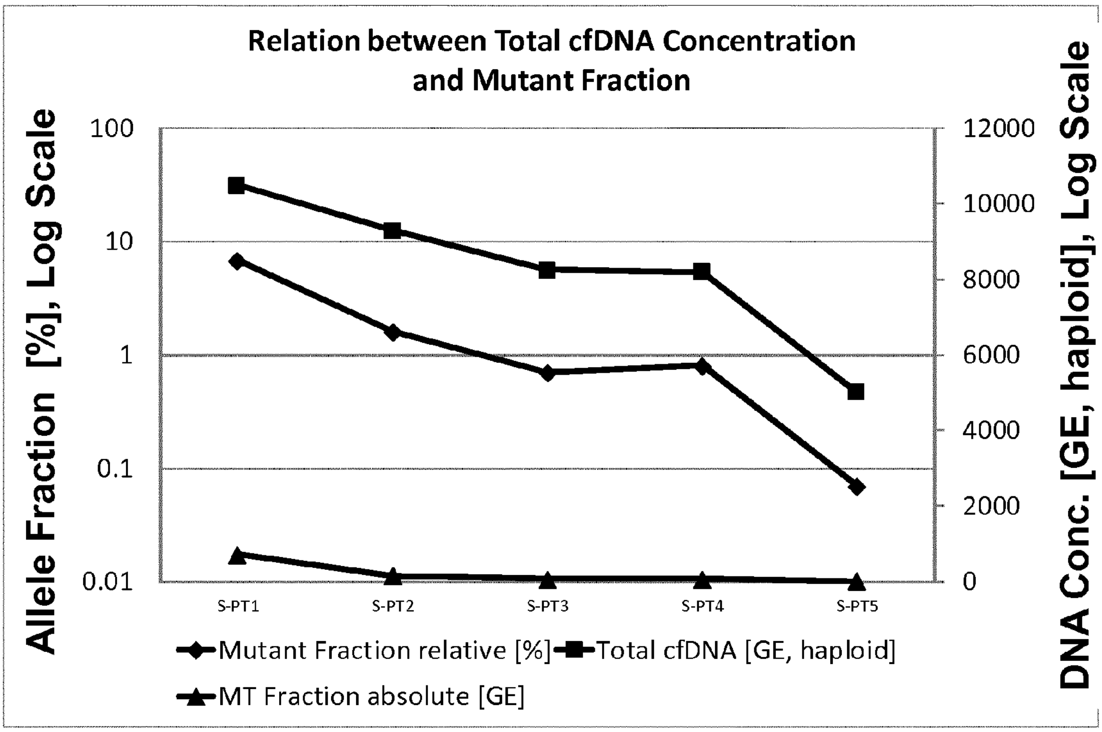
Relation between Total cfDNA Concentration
and Mutant Fraction
Allele Fraction [%], Log Scale
100
10
1
0.1
0.01
12000
10000
8000
6000
4000
2000
0
S-PT1
S-PT2
S-PT3
S-PT4
S-PT5
Mutant Fraction relative [%]
Total cfDNA [GE, haploid]
MT Fraction absolute [GE]
DNA Conc. [GE, haploid], Log Scale

**Fig. 5**

Europäisches Patentamt
European Patent Office
Office européen des brevets

# EUROPEAN SEARCH REPORT

Application Number
EP 19 16 9679

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/014920 A1 (UNIV JOHNS HOPKINS [US]; DIEHL FRANK [DE] ET AL.) 4 February 2010 (2010-02-04) * abstract; claim 25; example 6 * * paragraph [0007] - paragraph [0007] * * paragraph [0075] * * paragraph [0097] - paragraph [0101] * ----- | 1-12 | INV. C12Q1/6858 B01L3/00 |
| A | D. DRESSMAN ET AL: "Transforming single DNA molecules into fluorescent magnetic particles for detection and enumeration of genetic variations", PROCEEDINGS OF THE NATIONAL ACADEMY OF SCIENCES (PNAS), vol. 100, no. 15, 22 July 2003 (2003-07-22), pages 8817-8822, XP55560058, US ISSN: 0027-8424, DOI: 10.1073/pnas.1133470100 * page 8822, column 1, paragraph 2 * ----- | 1-12 | |
| A | PHENIX-LAN QUAN ET AL: "dPCR: A Technology Review", SENSORS, vol. 18, no. 4, 20 April 2018 (2018-04-20), page 1271, XP55624848, DOI: 10.3390/s18041271 * page 16 - page 20 * ----- -/-- | 1-12 | TECHNICAL FIELDS SEARCHED (IPC) C12Q B01L |

The present search report has been drawn up for all claims

1

| Place of search | Date of completion of the search | Examiner |
|---|---|---|
| Munich | 23 September 2019 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 16 9679

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | G. CZILWIK ET AL: "Rapid and fully automated bacterial pathogen detection on a centrifugal-microfluidic LabDisk using highly sensitive nested PCR with integrated sample preparation", LAB ON A CHIP, vol. 15, no. 18, 1 January 2015 (2015-01-01), pages 3749-3759, XP055559936, ISSN: 1473-0197, DOI: 10.1039/C5LC00591D * the whole document * ----- | 1-12 | |
| A | CHENG ZULE ET AL: "An emulsion digital PCR quantitative method based on microbeads and micropillar array chip", 2017 19TH INTERNATIONAL CONFERENCE ON SOLID-STATE SENSORS, ACTUATORS AND MICROSYSTEMS (TRANSDUCERS), IEEE, 18 June 2017 (2017-06-18), pages 575-578, XP033130821, DOI: 10.1109/TRANSDUCERS.2017.7994114 [retrieved on 2017-07-26] * the whole document * ----- | 1-12 | TECHNICAL FIELDS SEARCHED (IPC) |

The present search report has been drawn up for all claims

1

| Place of search | Date of completion of the search | Examiner |
|---|---|---|
| Munich | 23 September 2019 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 19 16 9679

This annex lists the patent family members relating to the patent documents cited in the above-mentioned European search report. The members are as contained in the European Patent Office EDP file on
The European Patent Office is in no way liable for these particulars which are merely given for the purpose of information.

23-09-2019

| Patent document cited in search report | Publication date | Patent family member(s) | Publication date |
|---|---|---|---|
| WO 2010014920 A1 | 04-02-2010 | CA 2732623 A1 | 04-02-2010 |
| | | EP 2315849 A1 | 04-05-2011 |
| | | JP 2011529691 A | 15-12-2011 |
| | | JP 2016104010 A | 09-06-2016 |
| | | US 2010041048 A1 | 18-02-2010 |
| | | WO 2010014920 A1 | 04-02-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9360526 B2


### Non-patent literature cited in the description


- **SIDRANSKY.** *Nat Rev Cancer,* 2002, vol. 2, 210-219
- **DRESSMAN et al.** *PNAS,* 2003, vol. 100 (15), 8817-8822
- *User guide. Dynabeads Antibody Coupling Kit,* 2012
- **SHAPIRO, HOWARD.** Practical Flow Cytometry. Wiley-Liss, 2003, 231-236
- **SHAPIRO, H.** Practical Flow Cytometry. Wiley-Liss, 2003, 236
- **GARCIA et al.** *Oncotarget,* 2018, vol. 9 (30), 21125-21126
- Elemental Analysis Manual for Food and Related Products, Version 2.0. *FDA,* 2015
- **REINARD, THOMAS.** Molekularbiologische Methoden 2.0. 2018, 85 f
- **SMITH.** *Environ Microbiol,* 2006, vol. 8 (5), 804-815
- **LEE et al.** *Scientific Reports,* 2018, vol. 8
- **ELNIFRO.** *Clin Microbiol Rev,* 2000, 559-570
- **DEBIASI.** *Arch Neurol,* 1999, vol. 56, 1215-1219
- **SHIN.** *Anticancer Res,* 2012, vol. 32, 163-168
- **WANG.** *Asian Pac J Cancer Prev,* 2015, vol. 16 (7), 3003-3007
- **REINARD.** *Molekularbiologische Methoden 2.0.,* 2018, 85 f
- **BEDU-ADDO.** *Pharmaceut Technol, Lyophilization,* 2004, 10-18
- **WITT.** *Eng Life Sci,* 2017, vol. 17, 953-958
- **GARCIA.** *Oncotarget,* 2018, vol. 9 (30), 21122-21131
- **SHAPIRO.** Practical Flow Cytometry. Wiley-Liss, 2003, 231-236
- **DIEHL.** BEAMing: single-molecule PCR on microparticles in water-in-oil emulsions. *Nature Methods,* 2006, vol. 3, 551-559
- **SHAPIRO.** FCS Express Software User Manual (De Novo Software. *FCS EXPRESS 6 RUO EDITION USERS MANUAL. [Software User Manual,* 2003
- **DE NOVO.** Software. *FCS EXPRESS 6 RUO EDITION USERS MANUAL. [Software User Manual,* 2016
- **DIEHL.** *Nat Med,* 2008, vol. 14 (9), 985-90
- **VERSCHOOR.** *Front Immunol,* 2015, vol. 6, 380
- **TABACK et al.** *Ann NY Acad Sci,* 2004, vol. 1022 (17-24), 19